# EUROPÄISCHE PATENTSCHRIFT

(11) **EP 2 121 234 B1**
(45) Veröffentlichungstag und Bekanntmachung des Hinweises auf die Patenterteilung: **21.06.2017**
(21) Anmeldenummer: 08706014.1
(22) Anmeldetag: 08.02.2008
(51) Int. Cl.: G01N 33/00, B23K 9/32

(54) **VORRICHTUNG UND VERFAHREN ZUR SCHUTZGASMESSUNG**
DEVICE AND METHOD FOR SHIELDING GAS MEASUREMENT
DISPOSITIF ET PROCÉDÉ DE MESURE DE GAZ PROTECTEUR

(30) Priorität: 22.02.2007 AT 2792007
(43) Veröffentlichungstag der Anmeldung: 25.11.2009
(73) Patentinhaber: Fronius International GmbH, 4643 Pettenbach (AT)
(72) Erfinder: EGLSEDER, Erich, A-4560 Kirchdorf/Krems (AT); LEONHARTSBERGER, Andreas, A-4490 St. Florian/Linz (AT)
(74) Vertreter: Sonn & Partner Patentanwälte
(86) Internationale Anmeldenummer: PCT/AT2008/000044
(87) Internationale Veröffentlichungsnummer: WO 2008/101264

(56) Entgegenhaltungen:
- JP-A- 56 056 782
- JP-A- 56 109 187
- JP-A- 61 033 766
- US-A- 4 733 050
- US-A- 5 329 092
- US-A- 5 442 155
- US-A1- 2004 026 392
- US-A1- 2006 169 745

## Beschreibung

Die Erfindung betrifft eine Vorrichtung zum Messen eines bei einem Lichtbogenschweißprozess eingesetzten Schutzgases, indem das aus einer Gasdüse eines Brenners austretende Schutzgas analysiert wird, wobei zur Schutzgasanalyse zumindest ein Sensor in einer externen Messvorrichtung angeordnet ist.

Ebenso betrifft die Erfindung ein Verfahren zum Messen eines bei einem Lichtbogenschweißprozess eingesetzten Schutzgases, indem das aus einer Gasdüse eines Brenners austretende Schutzgas analysiert wird.

Aus dem Stand der Technik sind bereits mehrere Dokumente bekannt, welche eine Schutzgasmessung beschreiben.

So beschreibt die DD 251 305 A1 die Messung des Schutzgases bzgl. der Schutzwirkung für den Lichtbogen eines Schweißprozesses, wobei aufgrund der Messergebnisse eine Stabilisierung des Schweißvorganges und eine Qualitätssicherung ermöglicht wird. Nachteilig ist hierbei, dass keine exakte Beurteilung der Eigenschaften des Schutzgases erfolgt, da die Schutzgasverhältnisse aufgrund von Parameterabweichungen bestimmt werden, indem die Strom-/Spannungswerte gemessen werden.

Die Schutzgasmessung kann auch mit einem Farbindikator erfolgen, wie dies in der DD 239 744 A1 beschrieben ist. Dabei verändert der Farbindikator gemäß der Sauerstoffanteile im Gas entsprechend seine Farbe. Hierbei ist nachteilig, dass auf das Messergebnis relativ lange gewartet werden muss. Ebenso ist es erforderlich, den Farbindikator nach jeder Messung auszutauschen.

Die DE 26 01 251 A1 beschreibt die Messung der Schutzwirkung einer Lichtbogen-Gashülle, indem die Stickstoff-Oxid(NO)-Konzentration gemessen wird. Dazu wird das Gas für die Analyse durch eine Absaugpumpe aus einer Hülse um die Gasdüse abgesaugt. Durch eine derartige Absaugpumpe sowie die dazugehörigen Komponenten wird die Zugänglichkeit des Schweißbrenners beeinträchtigt. Ebenso ist nachteilig, dass durch ein derartiges Messverfahren der Gasverbrauch steigt, wodurch sich auch die Kosten erhöhen. Des Weiteren wird hierbei lediglich die durch den Lichtbogen erzeugte NO-Konzentration in der Umgebung des Gasschutzbereiches detektiert, wodurch die Messung lediglich eine sehr geringe Aussagekraft besitzt. Dies ist damit begründet, dass die Menge der NO-Konzentration von mehreren Schweißparametern, wie Lichtbogenlänge, Schweißstrom, usw. abhängt, welche bei der Messung nicht berücksichtigt werden.

Die JP 9076062 A beschreibt eine Schweißmethode, bei der die Sauerstoffkonzentration in der Nähe der Schweißstelle gemessen wird, um Schweißfehler zu vermeiden. Das Verfahren ist beim Verschweißen von Rohren anwendbar, indem ein Ring zwischen die zu verschweißenden Rohre eingesetzt wird und durch ein radiales Loch im Ring ein Sensor zur Messung der Sauerstoffkonzentration im Inertgas eingesetzt wird.

Die US 5,329,092 A betrifft eine Einrichtung zur Kalibrierung und Überwachung von Fertigungsrobotern insbesondere Schweißrobotern. Zur periodischen Überwachung des vom Schweißbrenner ausströmenden Schutzgases wird der Schweißbrenner in eine trichterförmige Öffnung einer Messeinrichtung eingebracht und der resultierende Gasdruck ermittelt. Im Falle der Ermittlung eines unzureichenden Drucks wird der Schweißbrenner in eine entsprechende Wartungsposition verfahren und die Gasdüse gereinigt bzw. ausgetauscht.

Die Aufgabe der vorliegenden Erfindung liegt darin, die Schutzwirkung des Schutzgases schnell, exakt und praxisnahe zu ermitteln und entsprechend effektiv auszuwerten. Die Nachteile des Standes der Technik sollen vermieden oder zumindest reduziert werden.

Die Aufgabe der Erfindung wird dadurch gelöst, dass der zumindest eine Sensor in einem bei einem realen Schweißprozess im Wesentlichen dem Abstand zwischen dem Brenner und einem Werkstück entsprechenden Abstand zum Brenner positioniert ist, so dass der Austritt und die Wirkung des Schutzgases wie bei einem realen Schweißprozess simulierbar ist, und zur Beurteilung des Zustandes der Verschleißteile des Brenners zumindest ein Sensor zur Messung zumindest einer Gaseigenschaft des Schutzgases ausgebildet ist und mit einer Auswerteeinheit und über diese mit einem Schweißgerät verbunden ist, wobei zumindest ein Sensor durch einen Sauerstoffsensor gebildet ist. Daraus resultieren äußerst realistische Messresultate zur weiteren Verwendung. Ebenso ist von Vorteil, dass die Messvorrichtung nicht dem Schweißprozess ausgesetzt ist, wodurch die Gefahr einer Verschmutzung und/oder einer Beschädigung der Messvorrichtung sehr gering ist. Durch die Verbindung zumindest eines Sensors mit einer Auswerteeinheit und über diese mit einem Schweißgerät können aufgrund der Messergebnisse die Gaseigenschaften für die folgenden Schweißprozesse optimal abgestimmt werden. Ebenso kann die Gasmengenzufuhr den Umständen angepasst werden, so dass die exakte Menge des erforderlichen Schutzgases zur Verfügung gestellt wird. Dadurch werden auch Gaskosten eingespart. Dadurch, dass zumindest ein Sensor zur Messung zumindest einer Gaseigenschaft des Schutzgases ausgebildet ist sind Rückschlüsse auf die zu erwartende Qualität der Schweißnähte möglich.

Wenn die Messvorrichtung mit zumindest einem Sensor für die Schutzgasmessung mit einer Reinigungsvorrichtung für die Verschleißteile des Brenners kombiniert ist, muss in den Schweißpausen nur eine Station angefahren werden, wodurch Zeit eingespart werden kann.

Von Vorteil ist auch, wenn zumindest ein Sensor in der Messvorrichtung in seiner Lage veränderbar angeordnet ist, da dadurch unterschiedliche Schweißnahtpositionen entsprechend dem Werkstück simuliert oder Werkstückkonturen beim Sensor nachgebildet werden können (Kehlnaht, Ecknaht, Fallnaht, Überkopfnaht,...). Dadurch ist ein noch exakterer Vergleich zur Schweißung in der Praxis realisierbar.

Wenn eine Vorrichtung zur Aufnahme zumindest eines Sensors vorgesehen ist, kann die Messung des Schutzgases einfacher automatisiert werden und es können dadurch noch exaktere Messergebnisse erhalten werden. Dadurch können äußere Einflüsse im Bereich der Schweißnaht, wie ein Luftzug oder bauteilbedingte Gasströmungstoleranzen, berücksichtigt werden.

Dabei ist die Aufnahmevorrichtung vorzugsweise vom Schweißbrenner aufnehmbar und erfolgt die Schutzgasmessung entlang der tatsächlichen Werkstückkontur.

Wenn die Aufnahmevorrichtung einen Speicher zum Speichern der Messresultate aufweist und der Speicher beim Zurückstellen der Aufnahmevorrichtung in die Messvorrichtung mit der Auswerteeinheit verbunden ist, können die Messresultate automatisch in die Auswerteeinheit übertragen werden.

Dies kann auch dadurch geschehen, dass die Aufnahmevorrichtung ein Funkmodul zur drahtlosen Übertragung der Messresultate an die Auswerteeinheit aufweist.

Die Aufnahmevorrichtung kann an den Abstand zwischen Schweißbrenner und Werkstück gemäß einem Schweißprozess angepasst sein.

Wenn um zumindest einen Sensor eine vorzugsweise kreisförmige Einrichtung zur Führung von Druckluft angeordnet ist, kann der Sensor automatisch mit der Druckluft gereinigt werden. Ebenso kann dadurch eine einfache und schnelle Methode realisiert werden, die Gaseigenschaften der gesamten Schutzgashülle, insbesondere dessen Randbereich, zu messen.

Dabei weist die Druckluftführungseinrichtung vorzugsweise eine Druckluftkammer auf, welche mit einem Anschluss für die Druckluft versehen ist.

Wenn die Druckluftführungseinrichtung einen beweglichen Ring mit einer Auslassöffnung aufweist, durch welche die Druckluft aus der Druckluftkammer mit einem definierten Druck in Richtung des zumindest einen Sensors austritt, kann der Randbereich der Schutzgashülle gemessen werden, auch wenn der Brenner fix, beispielsweise auf einem Brennerschlitten, befestigt ist und keine Drehbewegung ausführen kann.

Wenn um zumindest einen Sensor zumindest eine Absaugöffnung angeordnet ist, welche mit einer Einrichtung zum Absaugen des Schutzgases verbunden ist, wird erreicht, dass keine Restgase in der unmittelbaren Umgebung des Sensors verbleiben, durch welche das Messergebnis verfälscht werden könnte.

Wenn die Messvorrichtung mit einer Abdeckung für zumindest einen Sensor versehen ist, kann der Sensor vor Verschmutzung geschützt werden und eine Beeinflussung des Messresultats durch die Verschmutzung vermieden werden.

Des Weiteren wird die Aufgabe der Erfindung auch durch ein oben genanntes Verfahren gelöst, wobei der Brenner über einer externen Messvorrichtung mit zumindest einem Sensor zur Schutzgasanalyse positioniert wird, der Abstand zwischen dem Brenner und zumindest einem Sensor im Wesentlichen entsprechend einem Abstand zwischen dem Brenner und einem Werkstück bei einem realen Schweißprozess gewählt wird, und zur Analyse das Schutzgas wie bei einem Schweißprozess aus der Gasdüse ausgeströmt wird, und mit zumindest einem Sensor zumindest eine Gaseigenschaft des Schutzgases gemessen und in einer mit dem Sensor verbundenen Auswerteeinheit ausgewertet wird, sodass der Austritt und die Wirkung des Schutzgases wie bei einem realen Schweißprozess simuliert wird, wobei als Gaseingenschaft des Schutzgases der Sauerstoffanteil des Schutzgases gemessen und ausgewertet wird, und automatisch auf Verunreinigungen der Verschleißteile des Brenners rückgeschlossen wird. Durch das Verfahren resultieren äußerst realistische Messresultate, welche sehr schnell von der Auswerteeinheit ausgewertet werden können und zur weiteren Verwendung zur Verfügung stehen, wie beispielsweise dem Schweißgerät. Dadurch, dass der Sauerstoffanteil des Schutzgases gemessen und ausgewertet wird, kann automatisch auf Verunreinigungen der Verschleißteile des Brenners rückgeschlossen werden. Dadurch kann ohne aufwendige optische Methoden der Verschmutzungsgrad erkannt werden. Somit wird ein Wechsel bzw. eine Reinigung der Verschleißteile nur durchgeführt, wenn dies wirklich erforderlich ist.

Wenn die Wirkung des Schutzgases im Zentrum einer Schutzgashülle ermittelt wird, erfolgt die Messung im selben Punkt der Schutzgashülle, in dem der Lichtbogen auf das Werkstück trifft.

Dabei kann zur Messung der Gaseigenschaft der Mittelpunkt eines Sensors in einer verlängerten Achse des Brenners bzw. eines darin verlaufenden Schweißdrahtes positioniert werden.

Gemäß einem weiteren Merkmal der Erfindung ist vorgesehen, dass die Wirkung des Schutzgases im Randbereich einer Schutzgashülle durch die Messung zumindest einer Gaseigenschaft des Schutzgases durch zumindest einen Sensor ermittelt wird. Auf diese Weise können Konzentrationsverteilung bestimmt werden. Ebenso ist die Erkennung einseitiger Verschmutzungen, welche evtl. für den gegebenen Schweißprozess (Schweißrichtung, Schweißposition) nicht oder schon relevant sind, erkennbar.

Zur Messung der Gaseigenschaft kann die Schutzgashülle mit Druckluft definiert verschoben werden, so dass der Randbereich der Schutzgashülle über den zumindest einen Sensor verschoben wird.

Dabei kann die Druckluft aus einer Auslassöffnung ausgeströmt werden und der Druck schrittweise erhöht werden.

Ebenso kann die auströmende Druckluft um die Schutzgashülle rotiert werden, so dass Schritt für Schritt der gesamte Teilbereich der Schutzgashülle über den zumindest einen Sensor verschoben wird.

Ebenso kann der Brenner im Wesentlichen kreisförmig um einen Sensor bewegt werden, wobei der Mittelpunkt der kreisförmigen Bewegung durch die Lage des Sensors gebildet wird.

Dabei können mit dem Brenner mehrere kreisförmige Bewegungen mit unterschiedlichen Radien um den Sensor durchgeführt werden.

Alternativ dazu oder zusätzlich kann der Brenner im Wesentlichen kreuzförmig um einen Sensor bewegt werden, wobei der Mittelpunkt der kreuzförmigen Bewegung durch den Sensor gebildet wird.

Wenn ein Sensor zur Messung mehrerer Gaseigenschaften herangezogen wird, ist die Messung nur an einer Position erforderlich und kann daher schnell durchgeführt werden.

Wenn hingegen für jede Eigenschaft des Schutzgases ein eigener Sensor herangezogen wird, können exaktere Messergebnisse erzielt werden, da die einzelnen Sensoren auf die zu messende Gaseigenschaft genauer abgestimmt werden können.

Dabei kann ein Sensor durch einen Sauerstoffsensor gebildet werden.

Wird eine Messung der Gaseigenschaften nach einer definierten Anzahl geschweißter Schweißnähte durchgeführt, wird erreicht, dass durch die periodischen Messungen zwischen den Schweißprozessen der Schweißprozess nicht durch die Messungen beeinflusst wird.

Ebenso kann die Messung nach einer Reinigung der Verschleißteile des Brenners durchgeführt werden, wodurch eine Kontrolle bzw. Anpassung der Gaseigenschaften an den folgenden Schweißprozess gegeben ist. Ebenso wird die Qualität der Schutzgaswirkung schon vor dem nächsten Schweißstart beurteilt.

Auch kann die Messung vor und nach einer Reinigung der Verschleißteile des Brenners durchgeführt werden, wodurch die Wirkung der Reinigung beurteilt werden kann. Demnach wird gegebenenfalls ein zweiter Reinigungsdurchgang durchgeführt bzw. ein Wechsel der Gasdüse angeordnet.

Gemäß einem weiteren Merkmal der Erfindung ist vorgesehen, dass die aktuell ermittelten Gaseigenschaften mit denen in einem Speicher der Auswerteeinheit in Form von hinterlegten und auf den Schweißprozess abgestimmten Referenzwerten verglichen werden. Dadurch kann eine Überprüfung der eingestellten Parameter für das Schutzgas am Schweißgerät durchgeführt werden.

Wird ein Ausgangspunkt des Brenners, der so genannte Tool Center Point, aufgrund des Zusammenhangs zwischen der Position des Brenners der gemessenen Schutzgaswirkung und der dazugehörigen Referenzwerte kontrolliert, kann die Position der Schweißnaht immer exakt eingehalten werden.

Vorteilhafterweise werden Daten zwischen der Auswerteeinheit und dem Schweißgerät ausgetauscht, wodurch Änderungen der Gaseigenschaften aufgrund der Messergebnisse unmittelbar nach der Messung, also noch vor dem folgenden Schweißprozess, durchgeführt werden können.

Wenn die ermittelten Gaseigenschaften in der Auswerteeinheit nach jeder Messung gespeichert werden, so dass eine Trendanalyse ermöglicht wird, können gezielte Aussagen darüber getroffen werden, wann Verschleißteile, wie die Gasdüse, ausgetauscht werden müssen. Ebenso kann dadurch eine Abschätzung getroffen werden, bis zu welchem Verschleißzustand die Verschleißteile noch wirkungsvoll eingesetzt werden können.

Wenn eine mit zumindest einem Sensor verbundene Aufnahmevorrichtung vom Schweißbrenner aufgenommen wird und die tatsächliche Werstückkontur mit einem Abstand zwischen Schweißbrenner und Werkstück gemäß einem Schweißprozess abgefahren wird, können äußere Einflüsse im Bereich der Schweißnaht, wie ein Luftzug oder Bauteil-bedingte Gasströmungstoleranzen, berücksichtigt werden.

Vorteilhafterweise wird die Werkstückkontur mit der Geschwindigkeit gemäß einem Schweißprozess abgefahren. Dies kommt den realen Bedingungen während des Schweißprozesses nahe.

Wenn die Resultate der Schutzgasmessung in einem Speicher in der Aufnahmevorrichtung gespeichert werden und beim Zurückstellen der Aufnahmevorrichtung in die Messvorrichtung die Messresultate automatisch von diesem Speicher in die Auswerteeinheit übertragen werden, oder die Resultate über ein in der Aufnahmevorrichtung angeordnetes Funkmodul zur Auswerteeinheit übertragen werden, stehen die Messergebnisse der Auswerteeinheit im Wesentlichen sofort zur Verfügung.

Vorteilhafterweise werden aufgrund der Resultate der Auswerteeinheit automatisch Rückschlüsse auf die Qualität der durchgeführten Schweißprozesse gezogen und wird die Qualität der Gaseigenschaften für die folgenden Schweißprozesse kontrolliert. Vorteilhaft ist hierbei, dass die Messung der Gaseigenschaften in einer Messvorrichtung sehr praxisnahe ist, wodurch eine sehr hochwertige Beurteilung der Gaseigenschaften in Bezug auf die Schweißnaht gewährleistet werden kann. Somit kann eine exakte Qualitätssicherung durchgeführt werden. Ebenso ist von Vorteil, dass mit der Auswertung der Gaseigenschaften genau die Eigenschaften analysiert werden, die unmittelbar für die Qualität der Schweißnaht verantwortlich sind. Auch ist vorteilhaft, dass aufgrund der Gaseigenschaften der Zustand der Verschleißteile beurteilt werden kann und dass die Messung der Gaseigenschaften mit einem sehr geringen Verbrauch des Schutzgases möglich ist. Von Vorteil ist des Weiteren, dass es zu keinen Überschreitungen von Grenzwerten der Schutzgaszusammensetzung kommen kann.

Dabei können Gaseigenschaften, wie Sauerstoffanteil, Stickstoffanteil, Kohlendioxidanteil, Gastemperatur, Ausströmgeschwindigkeit, Feuchtigkeitsanteil, Gasdruck, Gasdichte, Massendurchfluss bzw. Volumenstrom od. dgl., gemessen und ausgewertet werden. Dadurch werden schnelle Ergebnisse und somit schnelle Rückschlüsse auf die Qualität der Schweißnaht erzielt.

Weiters kann automatisch auf die Verwendung einer korrekten Gasdüse nach einem Verschleißteilewechsel rückgeschlossen werden, indem zumindest der Gasdruck und die Geschwindigkeit des Schutzgases gemessen und ausgewertet werden. Dadurch wird eine ständig gleich bleibende Qualität gewährleistet.

Weiters kann automatisch auf die Wirkung eines Kühlkreises des Brenners rückgeschlossen werden, indem die Temperatur des Schutzgases gemessen wird. Dadurch wird der Brenner vor Überhitzung geschützt.

Wenn automatisch auf die Temperatur des Schlauchpakets und des Brenners rückgeschlossen wird, indem die Temperatur des Schutzgases gemessen wird, können der Brenner und das Schlauchpaket vor zu hohen Temperaturen beim Start des Schweißprozesses geschützt werden. Auch werden dadurch die Verschleißteile geschont.

Wenn automatisch auf Poren in der zu schweißenden Schweißnaht und der geschweißten Schweißnaht rückgeschlossen wird, indem der Feuchtigkeitsanteil des Schutzgases gemessen wird, kann beurteilt werden, ob Poren gebildet wurden bzw. ob die Porenbildung innerhalb einer Toleranzgrenze liegt. Die Porenbildung ist ein Qualitätsmerkmal für die Schweißnaht.

Schließlich kann automatisch auf defekte Komponenten des Schutzgases rückgeschlossen werden, indem der Massendurchfluss der Komponenten des Schutzgases gemessen wird. Dadurch kann ein Gasverlust erkannt werden und können Kosten eingespart werden.

Die vorliegende Erfindung wird anhand der beigefügten, schematischen Zeichnungen näher erläutert. Darin zeigen:
Fig. 1 eine schematische Darstellung einer Schweißmaschine bzw. eines Schweißgerätes;
Fig. 2 eine perspektivische Ansicht einer Reinigungsvorrichtung mit der erfindungsgemäßen Messvorrichtung;
Fig. 3 die Positionierung des Schweißbrenners für die Schutzgasmessung;
Fig. 4 eine perspektivische Ansicht einer Reinigungsvorrichtung mit der erfindungsgemäßen Messvorrichtung und Druckluftführung;
Fig. 5 die Druckluftführung in Detailansicht; und
Fig. 6 die Positionierung des Schweißbrenners für die Schutzgasmessung in der Druckluftführung.

Einführend wird festgehalten, dass gleiche Teile des Ausführungsbeispiels mit gleichen Bezugszeichen versehen werden.

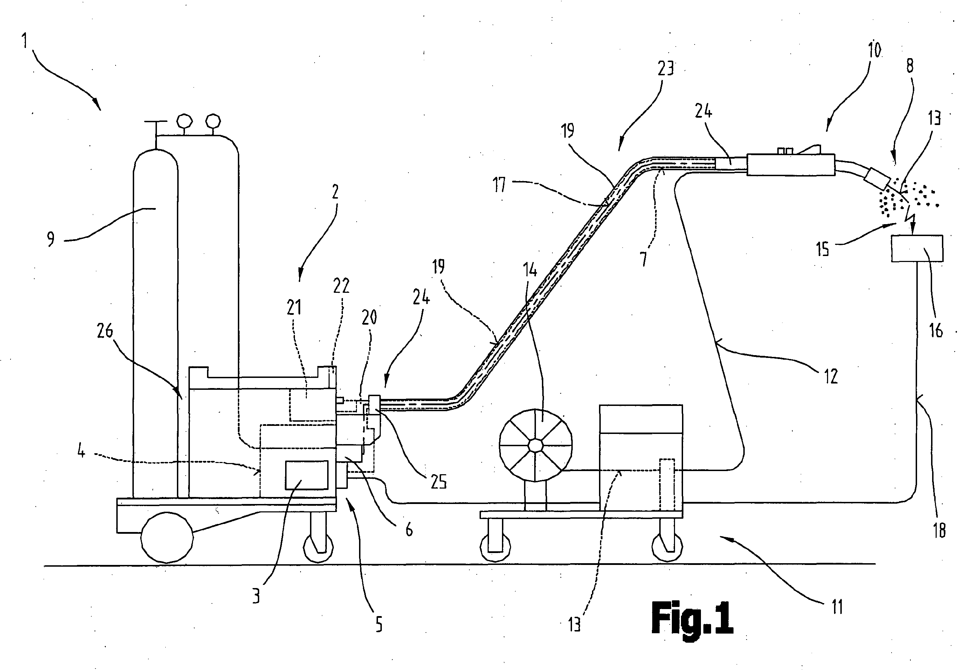

In Fig. 1 ist ein Schweißgerät 1 bzw. eine Schweißanlage für verschiedenste Prozesse bzw. Verfahren, wie z.B. MIG/MAG-Schweißen bzw. WIG/TIG-Schweißen oder Elektroden-Schweißverfahren, Doppeldraht/Tandem-Schweißverfahren, Plasma- oder Lötverfahren usw., gezeigt.

Das Schweißgerät 1 umfasst eine Stromquelle 2 mit einem Leistungsteil 3, einer Steuervorrichtung 4 bzw. Regelung und einem dem Leistungsteil 3 bzw. der Steuervorrichtung 4 zugeordneten Umschaltglied 5. Das Umschaltglied 5 bzw. die Steuervorrichtung 4 ist mit einem Steuerventil 6 verbunden, welches in einer Versorgungsleitung 7 für ein Gas 8, insbesondere ein Schutzgas, wie beispielsweise CO₂, Helium oder Argon u. dgl., zwischen einem Gasspeicher 9 und einem Schweißbrenner 10 bzw. einem Brenner angeordnet ist.

Zudem kann über die Steuervorrichtung 4 noch ein Drahtvorschubgerät 11, welches für das MIG/MAG-Schweißen üblich ist, angesteuert werden, wobei über eine Versorgungsleitung 12 ein Zusatzwerkstoff bzw. ein Schweißdraht 13 von einer Vorratstrommel 14 bzw. einer Drahtrolle in den Bereich des Schweißbrenners 10 zugeführt wird. Selbstverständlich ist es möglich, dass das Drahtvorschubgerät 11, wie es aus dem Stand der Technik bekannt ist, im Schweißgerät 1, insbesondere im Grundgehäuse, integriert ist und nicht, wie in Fig. 1 dargestellt, als Zusatzgerät ausgebildet ist.

Es ist auch möglich, dass das Drahtvorschubgerät 11 den Schweißdraht 13 bzw. den Zusatzwerkstoff außerhalb des Schweißbrenners 10 an die Prozessstelle zuführt, wobei hierzu im Schweißbrenner 10 bevorzugt eine nicht abschmelzende Elektrode angeordnet ist, wie dies beim WIG/TIG-Schweißen üblich ist.

Der Strom zum Aufbauen eines Lichtbogens 15, insbesondere eines Arbeitslichtbogens, zwischen der nicht abschmelzenden Elektrode, nicht dargestellt, und einem Werkstück 16 wird über eine Schweißleitung 17 vom Leistungsteil 3 der Stromquelle 2 dem Schweißbrenner 10, insbesondere der Elektrode, zugeführt, wobei das zu verschweißende Werkstück 16, welches aus mehreren Teilen gebildet ist, über eine weitere Schweißleitung 18 ebenfalls mit dem Schweißgerät 1, insbesondere mit der Stromquelle 2, verbunden ist und somit über den Lichtbogen 15 bzw. den gebildeten Plasmastrahl für einen Prozess ein Stromkreis aufgebaut werden kann.

Zum Kühlen des Schweißbrenners 10 kann über einen Kühlkreislauf 19 der Schweißbrenner 10 unter Zwischenschaltung eines Strömungswächters 20 mit einem Flüssigkeitsbehälter, insbesondere einem Wasserbehälter 21, verbunden werden, wodurch bei der Inbetriebnahme des Schweißbrenners 10 der Kühlkreislauf 19, insbesondere eine für die im Wasserbehälter 21 angeordnete Flüssigkeit verwendete Flüssigkeitspumpe, gestartet wird und somit eine Kühlung des Schweißbrenners 10 bewirkt werden kann.

Das Schweißgerät 1 weist weiters eine Ein- und/oder Ausgabevorrichtung 22 auf, über die die unterschiedlichsten Schweißparameter, Betriebsarten oder Schweißprogramme des Schweißgerätes 1 eingestellt bzw. aufgerufen werden können. Dabei werden die über die Ein- und/oder Ausgabevorrichtung 22 eingestellten Schweißparameter, Betriebsarten oder Schweißprogramme an die Steuervorrichtung 4 weitergeleitet und von dieser werden anschließend die einzelnen Komponenten der Schweißanlage bzw. des Schweißgerätes 1 angesteuert bzw. entsprechende Sollwerte für die Regelung oder Steuerung vorgegeben.

Weiters ist in dem dargestellten Ausführungsbeispiel der Schweißbrenner 10 über ein Schlauchpaket 23 mit dem Schweißgerät 1 bzw. der Schweißanlage verbunden. In dem Schlauchpaket 23 sind die einzelnen Leitungen vom Schweißgerät 1 zum Schweißbrenner 10 angeordnet. Das Schlauchpaket 23 wird über eine Kupplungsvorrichtung 24 mit dem Schweißbrenner 10 verbunden, wogegen die einzelnen Leitungen im Schlauchpaket 23 mit den einzelnen Kontakten des Schweißgerätes 1 über Anschlussbuchsen bzw. Steckverbindungen verbunden sind. Damit eine entsprechende Zugentlastung des Schlauchpaketes 23 gewährleistet ist, ist das Schlauchpaket 23 über eine Zugentlastungsvorrichtung 25 mit einem Gehäuse 26, insbesondere mit dem Grundgehäuse des Schweißgerätes 1, verbunden. Selbstverständlich ist es möglich, dass die Kupplungsvorrichtung 24 auch für die Verbindung am Schweißgerät 1 eingesetzt werden kann.

Grundsätzlich ist zu erwähnen, dass für die unterschiedlichen Schweißverfahren bzw. Schweißgeräte 1, wie beispielsweise WIG-Geräte oder MIG/MAG-Geräte oder Plasmageräte, nicht alle zuvor benannten Komponenten verwendet bzw. eingesetzt werden müssen. Hierzu ist es beispielsweise möglich, dass der Schweißbrenner 10 als luftgekühlter Schweißbrenner 10 ausgeführt werden kann.

Der Schweißbrenner 10 weist des Weiteren eine Gasdüse 27 auf, welche die Elektrode 13 umschließt und aus der während eines Schweißprozesses ständig das Schutzgas 8 ausströmt. Dadurch ist ununterbrochen um den Lichtbogen 15 herum eine Schutzgashülle 28 geschaffen, so dass der Lichtbogen 15 und der geschmolzene Metallbereich des Werkstücks 16 gegenüber der Außenatmosphäre 29 abgeschirmt werden kann. Dadurch wird erreicht, dass der Luftsauerstoff bzw. die Umgebungsluft während eines Schweißprozesses von der Schweißnaht ferngehalten wird und somit das flüssige Metall unter dem Lichtbogen 15 vor einer Oxidation, was die Schweißnaht schwächen würde, schützt. Das Ergebnis eines Schutzgasschweißprozesses ist somit eine qualitativ sehr hochwertige Schweißnaht. Hierzu wird üblicherweise die Ausströmmenge des Schutzgases 8 von der Bedienungsperson bzw. vom Schweißer rein quantitativ und nach der Erfahrung des Schweißers eingestellt, oder die Einstellung beruht auf Angaben in einer Gebrauchsanweisung, so dass auf diese Weise eine ausreichend gute Schutzgashülle 28 für den Lichtbogen 15 geschaffen werden kann. Das Ausmaß, mit dem der Lichtbogen 15 und der geschmolzene Metallbereich von der Außenatmosphäre 29 abgeschirmt wird, ändert sich in Abhängigkeit von der Schutzgasausströmmenge und dem Zustand und der Art, wie das Schutzgas 8 ausströmt bzw. wie die Verteilung des Schutzgases bei der Ausströmung aus der Gasdüse 27 aussieht. In der Praxis wird die Verteilung des Schutzgases 8 häufig durch in der Gasdüse 27 anhaftende Schweißspritzer oder Schmutz negativ beeinflusst, da dadurch vermehrt Umgebungsluft in die Schutzgashülle 28 gelangt und somit die Qualität der Schweißnaht verschlechtert wird. Daher ist es für die Qualität des Schweißprozesses vorteilhaft, den Sauerstoffanteil des Schutzgases 8 zu messen.

Erfindungsgemäß ist nun vorgesehen, dass die Schutzgasmessung über zumindest einen in einer Messvorrichtung 30 angeordneten Sensor 31 erfolgt. Dazu kann ein Sensor 31 derart ausgebildet sein, dass dieser die Eigenschaften des Schutzgases 8, wie beispielsweise den Sauerstoffanteil, erfasst und zur Auswertung an eine entsprechende Auswerteeinheit 32 weiterleitet.

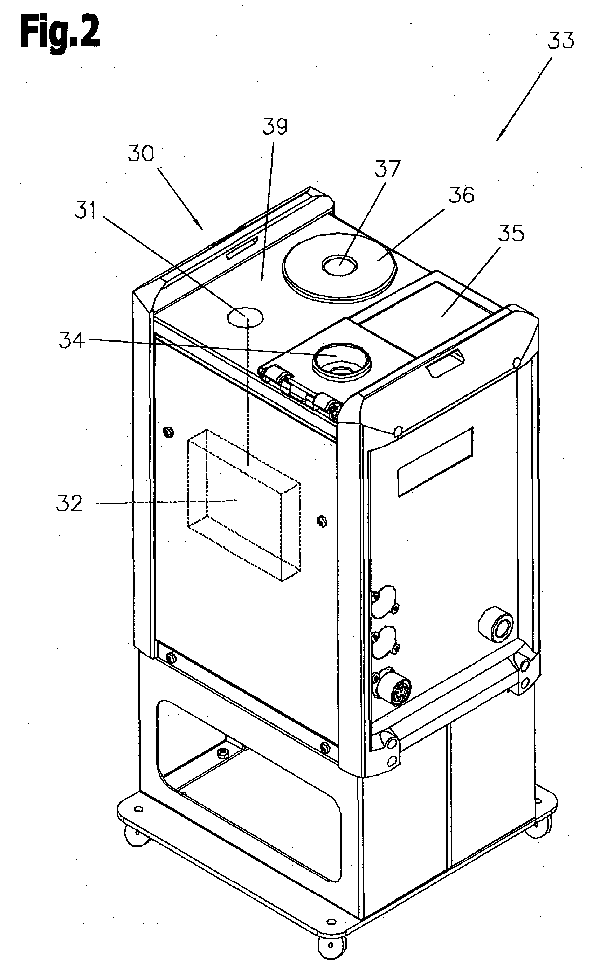

Bevorzugt wird die Messvorrichtung 30 in eine externe Reinigungsvorrichtung 33 gemäß Fig. 2 integriert, so dass der zumindest eine Sensor 31 in dieser angeordnet ist. Die Reinigungsvorrichtung 33 kann aber auch durch eine Servicestation od. dgl. für den Schweißbrenner 10 gebildet bzw. in solcher integriert sein. Durch eine derartige Zusammenführung werden die Schweißpausen für so genannte Serviceintervalle verkürzt, da nur eine Station, beispielsweise vom Roboter, angefahren werden muss.

Die Reinigungsvorrichtung 33 dient zur Reinigung des Schweißbrenners 10, wobei vorrangig die Verschleißteile des Schweißbrenners 10, insbesondere die Gasdüse 27, gereinigt werden. Die Reinigungsvorrichtung 33 kann eine Wanne 35 für die Reinigungsflüssigkeit aufweisen, in die der Schweißbrenner 10 eingetaucht wird, um die daran anhaftenden Metallspritzer abzukühlen. Neben der Flüssigkeitswanne 35 kann ein Nachfüllbehälter 34 angeordnet werden, über den die Wanne 35 mit Flüssigkeit versorgt werden kann. Hinter der Wanne 35 ist eine Spule 36 mit einer Öffnung 37 platziert. In die Öffnung 37 der Spule 36 wird der Schweißbrenner 10 nach dem Tauchen in die Wanne 35 platziert. Nach dem Versorgen der Spule 36 mit elektrischem Strom werden die Metallspritzer am Schweißbrenner 10 berührungslos entfernt. Die Verunreinigungen fallen in einen unter der Spule 36 angeordneten Abfallbehälter (nicht dargestellt). Da eine derartige Reinigungsvorrichtung 33 bereits aus dem Stand der Technik bekannt ist, wird nicht näher darauf eingegangen.

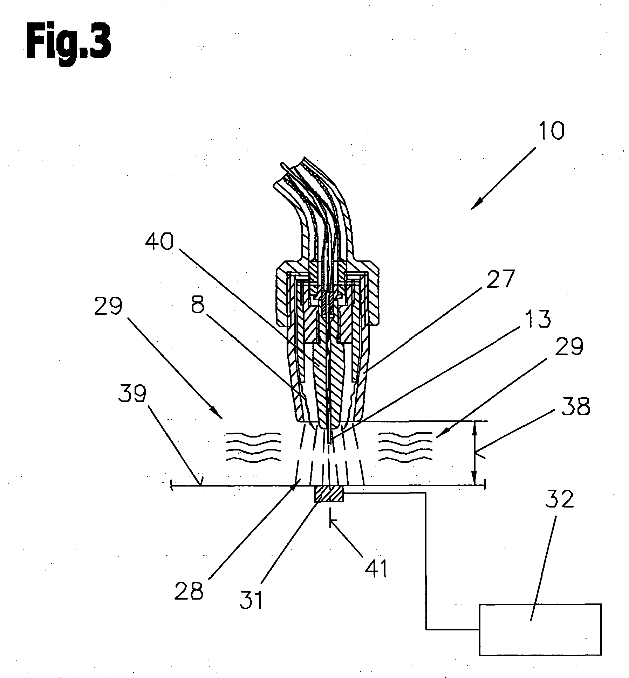

Gemäß der Erfindung wird nun die Reinigungsvorrichtung 33 mit dem zumindest einen Sensor 31 zur Messung der Eigenschaften des Schutzgases 8, wie Lufteinmischung, Temperatur, Strömungsgeschwindigkeit, Druck, Feuchtigkeit oder die Schutzgaszusammensetzung allgemein, erweitert. Dies hat den Vorteil, dass zusätzlich zu der ohnehin zyklisch durchgeführten Brenner- bzw. Gasdüsenreinigung auch die Eigenschaften des Schutzgases 8 gemessen werden. Aus dieser Messung kann dann bevorzugt ermittelt werden, welche Schweißnahtqualität bei den folgenden Schweißnähten zu erwarten ist bzw. die bereits geschweißten Schweißnähte aufweisen. Beispielsweise erfolgen derartige Messungen bzw. Reinigungen nach einer definierten Anzahl von Schweißnähten, wie zehn bis vierzig, nach einem definiertem Verbrauch des Schweißdrahttes, wie fünfzig bis hundert Meter, usw.. Somit würde es sich hierbei um eine so genannte Offline-Schutzgasmessung handeln. Da die Messungen bzw. Reinigungen jedoch innerhalb eines Prozesszyklus durchgeführt werden, kann durchaus von einer so genannten Online-Schutzgasmessung gesprochen werden. Die zur Messung zusätzlich benötigte Zeit liegt zwischen zwei und zehn Sekunden, welche hauptsächlich von der Anzahl der zu messenden Schutzgaseigenschaften pro Messvorgang abhängig ist. Damit die Messung im Wesentlichen den Verhältnissen entspricht, die während eines Schweißprozesses herrschen, wird der Schweißbrenner 10 im Wesentlichen so über dem Sensor 31 positioniert, dass sich ein Abstand 38 zwischen Sensor 31 und Schweißbrenner 10 einstellt, welcher dem Abstand zwischen Werkstück 16 und Schweißbrenner 10 entspricht, wie in Fig. 3 dargestellt: Dazu ist im Wesentlichen die Position des Schweißbrenners 10 normal zum Sensor 31, wobei der Schweißdraht 13 genau über dem Mittelpunkt des Sensors 31 angeordnet ist. Dadurch ist gewährleistet, dass der Sensor die Eigenschaften des Schutzgases 8 genau dort misst, wo bei einem Schweißprozess der Lichtbogen 15 auf das Werkstück 16 trifft. Dies wird dadurch erreicht, dass die Oberfläche 39 der Messvorrichtung 30 mit dem Sensor 31 eine Ebene bildet. Des Weiteren wird zur Messung exakt die Gasmenge aus der Gasdüse 27 ausgeströmt, die für die zuletzt geschweißten Schweißnähte herangezogen wurde bzw. die für die folgenden Schweißnähte herangezogen wird. Somit kann exakt überprüft werden, ob in Bezug auf den eingesetzten Brenner 10 bzw. die Gasdüse 27 die erforderliche Schutzwirkung des Schutzgases 8 für den Lichtbogen 15 gegeben war bzw. ob diese bei den folgenden Schweißnähten gegeben ist. Selbstverständlich wird dazu der Sensor 31 entsprechend kalibriert.

Bevorzugt wird vor einer Reinigung des Schweißbrenners eine Messung des Sauerstoffanteils in der Schutzgashülle 28 durchgeführt, so dass die eher zeitaufwendige Reinigung nur bei Bedarf ausgeführt wird. Hierzu kann beispielsweise für die Messung der Lufteinmischung bzw. des Sauerstoffanteils im Schutzgas 8 ein handelsüblicher Sauerstoffsensor eingesetzt werden. Sollte nun die Auswerteeinheit 32 einen zu hohen Sauerstoffanteil aufgrund des Messergebnisses erkennen, dass die erforderliche Schutzwirkung nicht gegeben ist, so wird bevorzugt eine Reinigung der Gasdüse 27, wie zuvor beschrieben, durchgeführt. Diese ist erforderlich, da aufgrund von anhaftenden Schweißspritzern in der Gasdüse 27 im austretenden Gasstrom Verwirbelungen entstehen, wodurch Luftsauerstoff in die Schutzgashülle 28 gelangt. Demnach werden die an der Innenseite der Gasdüse 27 anhaftenden Schweißspritzer bzw. der Spritzerring am Ende der Gasdüse 27 bei der Reinigung gelöst, so dass diese keine Verwirbelungen im ausströmenden Gas 8 mehr verursachen. Das heißt, das kein Luftsauerstoff mehr in die Schutzhülle 28 gelangt, wodurch die Schutzwirkung für den Lichtbogen 15 und in weiterer Folge die Qualität der Schweißnaht wieder den Anforderungen entspricht. Nach Durchführung der Reinigung kann beispielsweise nochmals eine Messung des Sauerstoffanteils durchgeführt werden, so dass das Ergebnis der Reinigung beurteilt werden kann. Ist das Ergebnis zufriedenstellend, können die folgenden Schweißprozesse bedenkenlos durchgeführt werden. Sollte das Ergebnis der Reinigung jedoch nicht die Erfordernisse erfüllen, so kann erneut eine Reinigung mit anschließender Kontrollmessung durchgeführt werden. Diese Abwechslung zwischen Reinigung und anschließender Kontrollmessung kann beispielsweise eine definierte Anzahl lang erfolgen. Wird jedoch durch diese Abwechslung kein zufriedenstellendes Ergebnis erzielt, muss die Gasdüse 27 von Hand gewechselt werden. Bei automatisierten Schweißsystemen kann die Ursache auch bei einem verstellten Tool Center Point liegen, wie später noch näher beschrieben wird. Durch die Messung des Sauerstoffanteils in der Schutzgashülle 28 kann somit ein Rückschluss auf den Grad der Verschmutzung der Gasdüse 27 gezogen werden.

Ebenso wie die Messung des Sauerstoffanteils kann auch die Temperatur, die Ausströmgeschwindigkeit, ein Feuchtigkeitsanteil, ein Druck, eine Dichte, ein Massendurchfluss bzw. Volumenstrom, der Massenanteil einer Komponente, usw. des Gases 8 durch den Sensor 31 gemessen werden. Im Allgemeinen wird durch die Schutzgasmessung erreicht, dass auf die Qualität der Schweißnaht rückgeschlossen werden kann bzw. diese vorhergesagt werden kann. Diese Qualität ist von mehreren Faktoren, wie der Kühlung des Brenners 10 oder der Zuleitung für das Schutzgas 8 abhängig. Welchen Einfluss diese einzelnen Faktoren auf die Qualität der Schweißnaht haben, kann eben durch die Messung der einzelnen Schutzgaseigenschaften kontrolliert werden.

Durch die Messung der Schutzgastemperatur kann auf die Kühlwirkung des Brenners 10 rückgeschlossen werden. Bei einer zu hohen gemessenen Schutzgastemperatur ist die erforderliche Kühlwirkung nicht gegeben, weshalb keine weiteren Schweißungen mehr durchgeführt werden können bzw. sollten. Aufgrund dieser Kontrolle ist also auch eine Überprüfung der Funktion aller Komponenten (Pumpe, Leitungen, etc.) des Kühlkreislaufs gegeben. Bevorzugt wird dem Schweißer der Fehler mitgeteilt oder entsprechend an der Ein- und/oder Ausgabevorrichtung 22 angezeigt, so dass der Fehler im Kühlkreislauf behoben werden kann, wobei gegebenenfalls noch weitere Messungen zum Eingrenzen des Fehlers erforderlich sein können.

Ebenso kann durch den gemessenen Feuchtigkeitsanteil im Schutzgas 8 auf die Anzahl oder Häufigkeit der Poren in der Schweißnaht rückgeschlossen werden. Hierzu sind bevorzugt entsprechende Referenzwerte in der Auswerteeinheit 32 oder der Steuervorrichtung 4 des Schweißgerätes hinterlegt, so dass beurteilt werden kann, ob der gemessene Feuchtigkeitsanteil gemäß den Anforderungen zulässig ist.

Aus der Messung der Ausströmgeschwindigkeit bzw. der Geschwindigkeitsverteilung kann auf den Zustand der Verteilerbohrungen, welche das Schutzgas 8 beim Eintritt in die Gasdüse 27 verteilen, rückgeschlossen werden. Die Ursache für eine Veränderung der Ausströmgeschwindigkeit sind häufig in der Gasdüse 27 anhaftende Schweißspritzer oder anderen Verunreinigungen, welche die Schutzwirkung für den Lichtbogen 15 beeinträchtigen.

Auch kann durch die Messung der einzelnen Schutzgaseigenschaften kontrolliert werden, ob sich entsprechend der Anwendung die richtige Gasdüse 27 am Schweißbrenner 10 befindet oder ob die verwendete Gasdüse 27 beschädigt bzw. während Schweißungen beispielsweise durch eine Kollision beschädigt wurde. Dies wird bevorzugt aufgrund der Messungen zumindest des Gasdrucks und der Geschwindigkeit ermittelt, welche unmittelbar das Ausströmverhalten des Schutzgases 8 aus der Gasdüse 27 beschreiben. Dies ist insbesondere für die unterschiedlichen Schweißnahtpositionen von Bedeutung, da durch die Geometrie, wie Durchmesser und Länge, der Gasdüse 27 die erforderliche Schutzwirkung für den Lichtbogen 15 bestimmt wird.

Durch die Messung der Schutzgaseigenschaften ist es auch in einfacher Weise möglich, die Zusammensetzung des Schutzgases 8 zu messen. Dadurch kann kontrolliert werden, ob für die geschweißten Schweißnähte bzw. die folgenden Schweißnähte auch die erforderliche Zusammensetzung des Schutzgases 8 verwendet wurde bzw. wird. Dementsprechend können auch die für die Schutzwirkung relevanten Gase, wie Kohlendioxid, Stickstoff, Argon, Helium, etc., gemessen werden.

Die Schutzgashülle 28 hat aber auch die Aufgabe, die unmittelbare Umgebung des Lichtbogens 15 vor der Umgebungsluft zu schützen, damit das Schmelzbad nicht oxidieren kann. Aus diesem Grund ist die Verteilung des Schutzgases 8, sprich das Ausströmverhalten, beim Austritt aus der Gasdüse 27 auch von Bedeutung.

Zuvor wurde beschrieben, wie die Gaseigenschaften am Auftreffpunkt des Lichtbogens 15 am Werkstück 16 gemessen werden. Um beispielsweise auch den Sauerstoffanteil im äußeren Bereich bzw. im Randbereich der Schutzhülle 28 zu messen, sind folgende Varianten möglich:
Es können zum Beispiel mehrere Sensoren 31 so angeordnet sein, dass die gesamte Schutzhülle 28 gleichzeitig gemessen werden kann. Bevorzugt werden jedoch mit dem an einem Roboter befestigten Brenner 10 bestimmte Bewegungen um den einen Sensor 31 ausgeführt. Dies kann beispielsweise durch eine senkrechte und eine waagrechte Bewegung, die sich am Sensor 31 überschneiden oder durch kreisförmige Bewegungen bzw. entlang einer Kurve um den Sensor 31 erfolgen. Dabei bildet beispielsweise der Sensor 31 den Mittelpunkt des Kreises wobei nach jeder vollständigen Umkreisung des Sensors 31 der Radius vergrößert wird, bis die Gaseigenschaften in der gesamten Schutzhülle 28 erfasst sind.

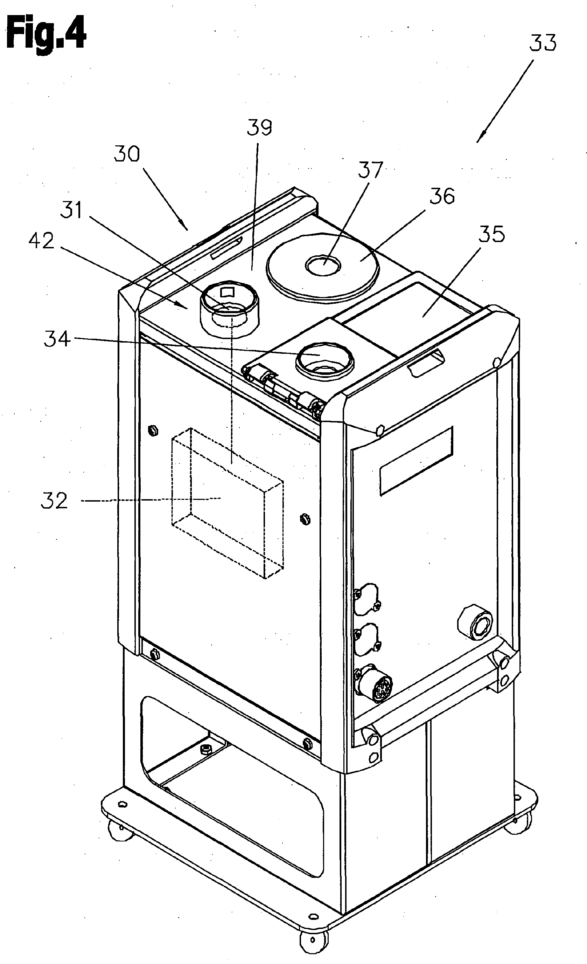

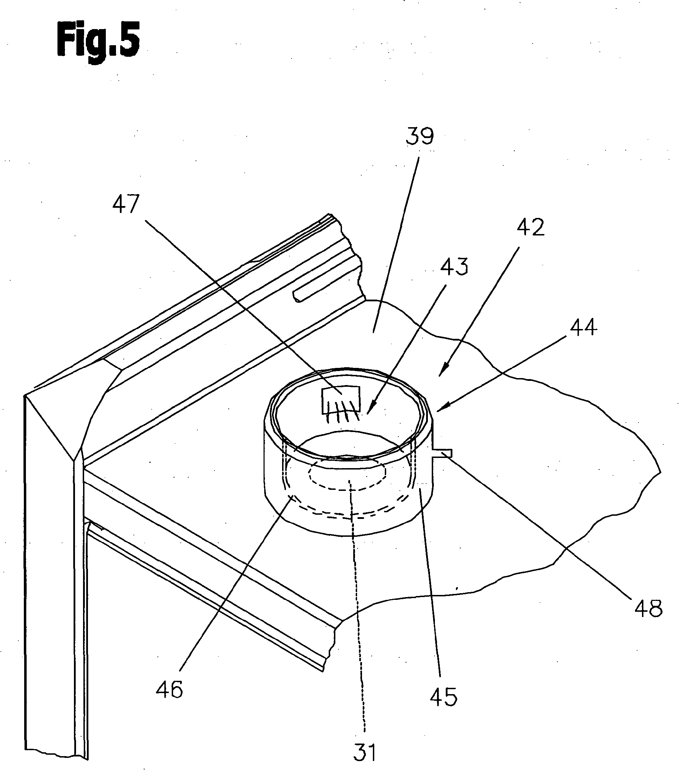

Eine weitere Möglichkeit, den Randbereich der Schutzgashülle 28 zu messen, ergibt sich durch den Einsatz einer Druckluftführung 42, wie in den Fig. 4 bis 6 dargestellt. Diese bewirkt, dass im Wesentlichen die Schutzgashülle 28 durch eine Druckluft 43 gezielt verschoben wird, so dass die Wirkung des Randbereichs der Schutzhülle 28 von dem in der Messvorrichtung 30 fix positionierten Sensor 31 gemessen werden kann. So kann bevorzugt der Randbereich der Schutzgashülle 28 vom Brenner 10 bzw. der Gasdüse 27 gemessen werden, welche keine Drehbewegungen ausführen können, wie zum Beispiel so genannte Brennerschlitten. Dazu ist die Druckluftführung 42 durch ein kreisförmiges Gehäuse 44, eine Druckluftkammer 45 und einen beweglichen Ring 46 mit einer Auslassöffnung 47 gebildet. In die Druckluftkammer 45, welche nach außen vom Gehäuse 44 und nach innen vom Ring 46 begrenzt wird, wird Druckluft 43 über einen entsprechenden Anschluss 48 zugeführt, so dass ein definierter Druck herrscht. Durch die Auslassöffnung 47 strömt dann die Druckluft 43 mit dem definierten Druck aus, so dass die Schutzgashülle 28 entsprechend verschoben wird. Bevorzugt wird das Ausströmen der Druckluft 43 durch ein in der Auslassöffnung 47 integriertes Ventil geregelt. Damit nun beispielsweise ein Profil der Sauerstoffverteilung in der Schutzgashülle 28 erstellt werden kann, ist der Ring 46 drehbar gelagert. Dadurch dreht sich die Auslassöffnung 47 rund um die Schutzgashülle 28, so dass der Sauerstoffanteil in jedem Bereich der Schutzgashülle 28 durch den fix montierten Sensor 31 erfasst werden kann. Hierbei ist es erforderlich, dass der Druck in der Druckluftkammer 45 schrittweise erhöht wird, so dass der Randbereich der Schutzgashülle 28 Schritt für Schritt über den Sensor 31 geschoben wird. Die Drehung des Rings 46 erfolgt dabei bevorzugt über einen Motor (nicht dargestellt). Somit kann die Rotationsgeschwindigkeit des Rings 46 bzw. der Auslassöffnung 47 an den Ausströmdruck der Druckluft 43 exakt angepasst werden, wobei der Ausströmdruck wiederum von der Ausströmgeschwindigkeit des Gases 8 abhängig ist. Diese Zusammenhänge sind bevorzugt in der Auswerteeinheit 32 entsprechend zum Typ des Schweißbrenners 10 bzw. der Gasdüse 27 hinterlegt.

Durch derartige Maßnahmen wird erreicht, dass die Gaseigenschaften auf der gesamten Fläche, die beim Auftreffen der Schutzhülle 28 auf das Werkstück 16 entsteht und somit den Schutzbereich für das Schmelzbad der Schweißnaht bildet, gemessen werden können. Somit können auch beispielsweise Verschmutzungen erkannt werden, die nur einseitig in der Gasdüse 27 vorhanden sind. Solche Verschmutzungen sind daher nur durch die Aufnahme eines Profils, wie zum Beispiel der Sauerstoffverteilung, erkennbar. Bei einer Mittelwertmessung der Sauerstoffkonzentration in der Schutzhülle 28 würde eine einseitige Verschmutzung mit hoher Wahrscheinlichkeit nicht erkennbar sein.

Selbstverständlich werden sämtliche Messergebnisse in der Auswerteeinheit 32 oder der Steuervorrichtung 4 gespeichert, so dass die Werte, Verteilungen und Profile der Schutzgashülle 28 bzw. deren Eigenschaften zu späteren Vergleichen zur Verfügung stehen. Ebenso können für sämtliche Schutzgaseigenschaften Referenzwerte hinterlegt sein, so dass eine unmittelbare Überprüfung der Messung erfolgen kann. Durch die Speicherung dieser Daten ist es auch möglich, eine so genannte Trendanalyse durchzuführen. Das heißt, das die Veränderung der Schutzhülle 28 bei zunehmender Einsatzdauer der Gasdüse 27 beobachtet wird. Aus diesen Ergebnissen heraus kann dann beispielsweise der Reinigungszyklus des Brenners 10 bzw. der Gasdüse 27 anhand der Wirkung der Schutzhülle 28 optimiert werden. Sprich, die Anzahl der Schweißnähte, die mit einer gereinigten Gasdüse 27 geschweißt werden können, erhöht sich beispielsweise von etwa zehn auf fünfzehn.

Ebenso werden bevorzugt die Messergebnisse auch direkt für die Einstellungen der Schweißparameter berücksichtigt. Beispielsweise derart, dass die Zusammensetzung des Schutzgases 8 mit der am Schweißgerät 1 eingestellten Zusammensetzung verglichen wird. Ebenso kann auf diese Art und Weise auch die Gasdurchflussmenge, die Ausströmgeschwindigkeit, etc. mit den eingestellten Werten abgeglichen bzw. korrigiert werden. Dies ist insbesondere dann von Vorteil, wenn neue Verschleißteile, wie Gasdüse 27, Kontaktrohr 40, etc., eingesetzt werden. Somit können Fehler in der Schutzhülle 28 sowie die aufgrund der Messungen rückschließbaren Fehler, wie zuvor beschrieben, im Vorfeld erkannt und behoben werden, so dass die Qualität der Schweißnaht nicht verschlechtert wird.

Um diese Vielzahl an Schutzgaseigenschaften messen zu können, werden in der Praxis im Wesentlichen mehrere Sensoren 31 herangezogen. Das heißt, dass im Wesentlichen mit je einem Sensor 31 nacheinander die entsprechende Gaseigenschaft gemessen wird. Beispielsweise erfolgt dies derart, dass der Sensor 31 nach der erfolgten Messung einer Schutzgaseigenschaft automatisch gewechselt wird. Dazu sind beispielsweise fünf Sensoren 31 an einem Rad befestigt (nicht dargestellt), das eine Drehbewegung ausführt, um den entsprechenden Sensor 31 an diejenige Position zu bewegen, dass sich dieser am Auftreffpunkt des Lichtbogens 15 befindet.

Eine weitere Möglichkeit der gleichzeitigen Erfassung mehrerer Messwerte kann auch dadurch bewerkstelligt werden, dass die notwendigen Sensoren 31 in einem Hohlraum unter der Oberfläche 39 der Messvorrichtung 30 angebracht sind. Dieser Hohlraum ist bevorzugt anstelle des in Fig. 3 dargestellten Sensors 31 angeordnet, wobei der Hohlraum eine oder auch mehrere Öffnungen in Richtung des Auftreffpunktes des Lichtbogens 15 aufweist. Durch diese Öffnungen gelangt das auftreffende Gas 8 zu den einzelnen Sensoren 31, die dann die einzelnen Schutzgaseigenschaften messen.

Selbstverständlich kann alternativ dazu die Messung der einzelnen Schutzgaseigenschaften (Sauerstoffanteil, Temperatur, Feuchtigkeit, Druck, ...) auch von einem einzigen Sensor 31, der gleichzeitig alle Schutzgaseigenschaften erfassen kann, durchgeführt werden.

Um für die zu simulierende Schweißnahtposition exakte Messergebnisse zu erhalten, können in der Messvorrichtung 30 beispielsweise bewegliche Teile enthalten sein. Diese können dann bevorzugt derart automatisiert bewegt werden, dass die zu simulierende Schweißnahtposition, wie z.B. eine Kehlnaht, entsteht. Selbstverständlich wird entsprechend dazu auch der Sensor 31 bewegt, so dass sich dieser exakt in der verlängerten Achse 41 der Elektrode 13 bzw. des Schweißdrahtes 13 befindet. Dies ist dementsprechend auch bei den zuvor beschriebenen Messungen der Fall. Da jedoch die Messergebnisse kaum abweichen, wenn der Schweißbrenner 10 im Wesentlichen normal zur Oberfläche 39 steht, ist diese Methode für die Praxis besser geeignet. Dies ist auch darauf zurückzuführen, dass diese Methode mit wesentlich weniger Aufwand und daher kostengünstiger realisierbar ist.

Um noch exaktere Messergebnisse bzw. um Messergebnisse, die den Umgebungsbedingungen des zu schweißenden Werkstücks 16 angepasst sind, zu erhalten, kann die Messvorrichtung 30 zusätzlich eine Aufnahmevorrichtung (nicht dargestellt) für den Sensor 31 beinhalten. Das heißt, dass der Schweißbrenner 10 so ausgebildet ist, dass dieser die Aufnahmevorrichtung, bevorzugt automatisiert, aufnehmen und fixieren kann, wobei anschließend von der Aufnahmevorrichtung der Sensor 31 aufgenommen und fixiert wird. Dazu ist selbstverständlich der Sensor 31 in einer entsprechenden Halterung angeordnet, so dass er von der Aufnahmevorrichtung aufgenommen werden kann.

Dieses Verfahren hat insbesondere den Vorteil, dass die Aufnahmevorrichtung entsprechend dem Abstand zwischen Schweißbrenner 10 und Werkstück 16 gemäß dem jeweiligen Schweißprozesse angepasst werden kann, indem beispielsweise mehrere Aufnahmevorrichtungen mit unterschiedlicher Länge in der Messvorrichtung 30 positioniert sind. Selbstverständlich kann die Länge der Aufnahmevorrichtung auch derart variiert werden, indem die Befestigungsposition am Brenner 10 entsprechend gewählt wird.

Nachdem also der Sensor 31 zur Messung der Gaseigenschaften am Schweißbrenner 10 befestigt ist, kann die zu schweißende Schweißnaht entsprechend entlang dieser Kontur abgefahren werden. Dabei wird dementsprechend Schutzgas 8 ausgeströmt, um die erforderlichen Eigenschaften, wie bereits zuvor beschrieben, messen zu können. Die hierbei erhaltenen Messresultate sind aufgrund der realistischen Messbedingungen sehr aussagekräftig, da zusätzliche Faktoren, wie Luftzug oder bauteilbedingte Gasströmungstoleranzen, für die Qualitätsbeurteilung berücksichtigt sind. Selbstverständlich könnten diese Faktoren auch bei der Messvorrichtung 30 simuliert werden, wobei eine derartige Nachbildung der realistischen Messbedingungen einen hohen Aufwand bedeuten würde.

Je nach Ausführung der Aufnahmevorrichtung können die Messresultate entweder in dieser zwischengespeichert werden oder direkt über ein Funkmodul an die Auswerteeinheit 32 übertragen werden. Im Fall, dass eine Zwischenspeicherung erfolgt, ist entsprechend ein Speicher in der Aufnahmevorrichtung integriert. Nachdem die Werkstückkontur bzw. die Werkstückkonturen abgefahren wurden, wird der Sensor 31 und die Aufnahmevorrichtung wieder an der Reinigungsvorrichtung 33 platziert. Damit nun die Messresultate aus dem Speicher ausgelesen werden können, wird beispielsweise beim Platzieren der Aufnahmevorrichtung eine Verbindung mit der Auswerteeinheit 32 hergestellt. Dabei kann die Verbindung zur Übertragung der Messresultate aus sämtlichen aus dem Stand der Technik bekannten Methoden durchgeführt werden.

Da die erfindungsgemäße Messvorrichtung 30 extern, wie beispielsweise in der Reinigungsvorrichtung 33, platziert ist, sind entsprechende Vorkehrungen gegen die Verschmutzung, insbesondere des Sensors 31, von Vorteil. Bevorzugt wird dies durch eine Abdeckung realisiert, welche automatisch nach einer Messung den Sensor 31 zudeckt, so dass dieser vor Staub, Schweißspritzern, usw. geschützt ist. Beispielsweise wird die Abdeckung durch eine Glasscheibe realisiert, welche durch eine Drehbewegung den Sensor 31 abdeckt bzw. für die Messung wieder freigibt. Gemäß der erfindungsgemäßen Ausführungsform mit der Druckluftführung 42 kann der Sensor 31 beispielsweise durch einen Deckel auf dem Gehäuse 44 abgedeckt werden.

Da der Sensor 31 nur periodisch für Messungen eingesetzt wird, können die Pausen für Reinigungszwecke des Sensors 31 genutzt werden. Bevorzugt erfolgt dies bei geschlossener Abdeckung und der Druckluft 43, wodurch eventuelle Schmutzreste aus der Gasdüse 27, die sich durch das ausströmende Gas 8 bei der Messung gelöst haben, weggeblasen werden. Da bei der Randbereich-Messung der Schutzgashülle 28 sowieso Druckluft 43 ausströmt, wird hierbei der Sensor 31 auch während der Messung automatisch gereinigt.

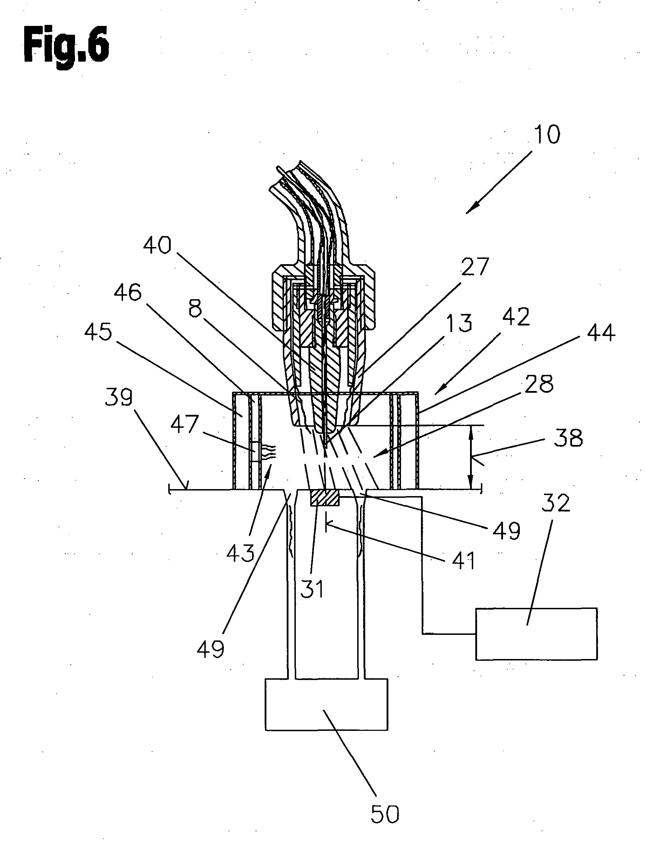

Dabei wird der Schmutz bevorzugt durch eine Absaugöffnung 49 um den Sensor 31 entfernt, wobei dies bevorzugt durch eine Absaugung 50 unterstützt wird, wie in Fig. 6 dargestellt. Des Weiteren kann die Absaugung 50 auch bevorzugt dazu verwendet werden, um das im Messbereich um den Sensor 31 angesammelte Schutzgas 8 abzusaugen. Somit wird das Messergebnis der folgenden Messungen nicht verfälscht.

Ebenso kann die Absaugung 50 auch während einer Messung aktiviert werden, so dass das ausströmende Schutzgas 8 die Außenatmosphäre 29 nicht verdrängen kann. Dies würde beispielsweise bewirken, dass bei Verwirbelungen kein Luftsauerstoff in die Schutzgashülle 28 eingesogen wird, sondern im Wesentlichen das Schutzgas 8 selbst, so dass das Messergebnis verfälscht wird.

Gemäß der Erfindung erfolgt die Messung der Schutzgaseigenschaften, wie beispielsweise die Messung des Sauerstoffanteils, im Wesentlichen derart, dass der Brenner 10 normal zur Oberfläche 39 und somit auch zum Sensor 31 steht, wobei auch der Abstand 38 des Brenners 10 zum Sensor 31 gemäß einem Schweißprozess gewählt wird. Bevorzugt wird die Erfindung bei automatisierten Schweißanlagen eingesetzt, das heißt, dass ein Roboter den Schweißbrenner 10 über den Sensor 31 positioniert. Dies bewerkstelligt der Roboter über den aus dem Stand der Technik bekannten Tool Center Point (TCP), welcher als Ausgangspunkt für die Bewegungen des Roboters dient. Daher ist es auch wichtig, dass der TCP stets korrekt ist. Aus der Praxis ist jedoch bekannt, dass sich durch eventuelle Kollisionen des Brenners 10 mit dem Werkstück 16 oder Ähnlichem oder durch Hitzeeinwirkung der TCP verstellen kann. Somit ist es von Vorteil, den TCP beispielsweise periodisch zu kontrollieren. Erfindungsgemäß ist dieses Feature durch die Messung einer Schutzgaseigenschaft realisiert. Dazu ist in der Auswerteeinheit 32 ein Referenzwert hinterlegt, der mit dem aktuell gemessenen Wert verglichen wird. Ergibt sich dabei eine gewisse Differenz außerhalb eines gewissen Toleranzbereiches, ist der TCP verstellt und muss korrigiert werden bzw. die Differenz von der Steuervorrichtung 4 oder der Robotersteuerung berücksichtigt werden. Bevorzugt erfolgt dies nach einer Reinigung des Brenners 10 bzw. der Gasdüse 27, so dass sichergestellt ist, dass die Abweichung der Messung nicht durch Verunreinigungen verursacht wurde.

Beispielsweise kann die Auswerteeinheit 32 auch durch die Steuerung 4 des Schweißgerätes 1 gebildet sein oder Teil einer übergeordneten Steuerung der Schweißanlage (Schweißgerät, Roboter, Drahtvorschub, usw.) sein. Des Weiteren können die Auswerteeinheit 32 und der Sensor 31 eine Einheit bilden.

Ebenso ist es denkbar, dass die Schutzgasmessung durch die Messvorrichtung 30 auch in einer Komponente der Schweißanlage, wie der Drahtvorschubeinheit oder dem Schlauchpaket, erfolgt. Hierbei handelt es sich dann um eine reine Online-Schutzgasmessung, wobei die Resultate nicht so praxisnahe sind, wie wenn die Messung erfindungsgemäß mit dem gleichen Abstand wie bei einem Schweißprozess erfolgt.

## Patentansprüche

1. Vorrichtung zum Messen eines bei einem Lichtbogenschweißprozess eingesetzten Schutzgases (8), indem das aus einer Gasdüse (27) eines Brenners (10) austretende Schutzgas (8) analysiert wird, wobei zur Schutzgasanalyse zumindest ein Sensor (31) in einer externen Messvorrichtung (30) angeordnet ist, wobei der zumindest eine Sensor (31) in einem bei einem realen Schweißprozess im Wesentlichen dem Abstand zwischen dem Brenner (10) und einem Werkstück (16) entsprechenden Abstand (38) zum Brenner (10) positioniert ist, so dass der Austritt und die Wirkung des Schutzgases (8) wie bei einem realen Schweißprozess simulierbar ist, und dass zur Beurteilung des Zustandes der Verschleißteile des Brenners (10) zumindest ein Sensor (31) zur Messung zumindest einer Gaseigenschaft des Schutzgases (8) ausgebildet und mit einer Auswerteeinheit (32) und über diese mit einem Schweißgerät (1) verbunden ist, **dadurch gekennzeichnet, dass** zumindest ein Sensor (31) durch einen Sauerstoffsensor (31) gebildet ist.

2. Messvorrichtung nach Anspruch 1, **dadurch gekennzeichnet, dass** die Messvorrichtung (30) mit zumindest einem Sensor (31) für die Schutzgasmessung mit einer Reinigungsvorrichtung (33) für die Verschleißteile des Brenners (10) kombiniert ist.

3. Messvorrichtung nach Anspruch 1 oder 2, **dadurch gekennzeichnet, dass** zumindest ein Sensor (31) in der Messvorrichtung (30) in seiner Lage veränderbar angeordnet ist.

4. Messvorrichtung nach einem der Ansprüche 1 bis 3, **dadurch gekennzeichnet, dass** um zumindest einen Sensor (31) eine vorzugsweise kreisförmige Einrichtung (42) zur Führung von Druckluft (43) angeordnet ist.

5. Messvorrichtung nach einem der Ansprüche 1 bis 4, **dadurch gekennzeichnet, dass** um zumindest einen Sensor (31) zumindest eine Absaugöffnung (49) angeordnet ist, welche mit einer Einrichtung (50) zum Absaugen des Schutzgases (8) verbunden ist.

6. Verfahren zum Messen eines bei einem Lichtbogenschweißprozess eingesetzten Schutzgases (8), indem das aus einer Gasdüse (27) eines Brenners (10) austretende Schutzgas (8) analysiert wird, wobei der Brenner (10) über einer externen Messvorrichtung (30) mit zumindest einem Sensor (31) zur Schutzgasanalyse positioniert wird, wobei der Abstand (38) zwischen dem Brenner (10) und zumindest einem Sensor (31) im Wesentlichen entsprechend einem Abstand (38) zwischen dem Brenner (10) und einem Werkstück (16) bei einem realen Schweißprozess gewählt wird, und dass zur Analyse das Schutzgas (8) wie bei einem Schweißprozess aus der Gasdüse (27) ausgeströmt wird, und mit zumindest einem Sensor (31) zumindest eine Gaseigenschaft des Schutzgases (8) gemessen und in einer mit dem Sensor (31) verbundenen Auswerteeinheit (32) ausgewertet wird, so dass der Austritt und die Wirkung des Schutzgases (8) wie bei einem realen Schweißprozess simuliert wird, **dadurch gekennzeichnet, dass** als Gaseigenschaft des Schutzgases (8) der Sauerstoffanteil des Schutzgases (8) gemessen und ausgewertet wird, und automatisch auf Verunreinigungen der Verschleißteile des Brenners (10) rückgeschlossen wird.

7. Messverfahren nach Anspruch 6, **dadurch gekennzeichnet, dass** die Wirkung des Schutzgases (8) im Zentrum einer Schutzgashülle (28) ermittelt wird, wobei zur Messung der Gaseigenschaften der Mittelpunkt eines Sensors (31) in einer verlängerten Achse (41) des Brenners (10) bzw. eines darin verlaufenden Schweißdrahtes (13) positioniert wird.

8. Messverfahren nach Anspruch 6 oder 7, **dadurch gekennzeichnet, dass** die Wirkung des Schutzgases (8) im Randbereich einer Schutzgashülle (28) durch die Messung zumindest einer Gaseigenschaft des Schutzgases (8) durch zumindest einen Sensor (31) ermittelt wird.

9. Messverfahren nach Anspruch 8, **dadurch gekennzeichnet, dass** der Brenner (10) im Wesentlichen kreisförmig um einen Sensor (31) bewegt wird, wobei der Mittelpunkt der kreisförmigen Bewegung durch die Lage des Sensors (31) gebildet wird.

10. Messverfahren nach einem der Ansprüche 6 bis 9, **dadurch gekennzeichnet, dass** ein Sensor (31) zur Messung mehrerer Gaseigenschaften herangezogen wird.

11. Messverfahren nach einem der Ansprüche 6 bis 9, **dadurch gekennzeichnet, dass** für jede Eigenschaft des Schutzgases (8) ein eigener Sensor (31) herangezogen wird.

12. Messverfahren nach einem der Ansprüche 6 bis 11, **dadurch gekennzeichnet, dass** die aktuell ermittelten Gaseigenschaften mit denen in einem Speicher der Auswerteeinheit (32) in Form von hinterlegten und auf den Schweißprozess abgestimmten Referenzwerten verglichen werden.

13. Messverfahren nach Anspruch 12, **dadurch gekennzeichnet, dass** ein Ausgangspunkt des Brenners (10), der so genannte Tool Center Point (TCP), aufgrund des Zusammenhangs zwischen der Position des Brenners (10), der gemessenen Schutzgaswirkung und der dazugehörigen Referenzwerte kontrolliert wird.

14. Messverfahren nach einem der Ansprüche 6 bis 13, **dadurch gekennzeichnet, dass** automatisch auf die Verwendung einer korrekten Gasdüse (27) nach einem Verschleißteilewechsel rückgeschlossen werden kann, indem zumindest der Gasdruck und die Geschwindigkeit des Schutzgases (8) gemessen und ausgewertet werden.

15. Messverfahren nach einem der Ansprüche 6 bis 14, **dadurch gekennzeichnet, dass** automatisch auf Poren in der zu schweißenden Schweißnaht und der geschweißten Schweißnaht rückgeschlossen wird, indem der Feuchtigkeitsanteil des Schutzgases (8) gemessen wird.

## Claims

1. An arrangement for measuring a protective gas (8) used in an arc-welding process by analyzing the protective gas (8) escaping from a gas nozzle (27) of a torch (10), wherein at least one sensor (31) is arranged in an external measuring device (30) for protective-gas analysis, wherein the at least one sensor (31) is positioned at a distance (38) from the torch (10) which substantially equals the distance between the torch (10) and a workpiece (16) in an actual welding process so that the escape and effect of the protective gas (8) of an actual welding process can be simulated, and that at least one sensor (31) for measuring at least one gas property of a protective gas (8) is connected to an evaluation unit (32) and via the latter to a welding device (1) so as to assess the state of the wearing parts of the torch (10), **characterized in that** at least one sensor (31) is comprised of an oxygen sensor (31).

2. The measuring arrangement according to claim 1, **characterized in that** the measuring device (30) is combined with at least one sensor (31) for protective-gas measurement with a cleaning unit (33) for the wearing parts of the torch (10).

3. The measuring arrangement according to claim 1 or 2, **characterized in that** at least one sensor (31) is arranged in the measuring device (30) in a position-changeable manner.

4. The measuring arrangement according to any one of claims 1 to 3, **characterized in that** a preferably circular means (42) for guiding pressurized air (43) is arranged about at least one sensor (31).

5. The measuring arrangement according to any one of claims 1 to 4 **characterized in that** at least one suction opening (49) is arranged about at least one sensor (31), which suction opening (49) is connected to a means (50) for sucking off the protective gas (8).

6. A method of measuring a protective gas (8) used in an arc-welding process by analyzing the protective gas (8) escaping from a gas nozzle (27) of a torch (10), wherein the torch (10) is positioned above an external measuring device (30) with at least one sensor (31) for protective-gas analysis, wherein the distance (38) between the torch (10) and the at least one sensor (31) is selected to be substantially the same as the distance (38) between the torch (10) and a workpiece (16) in an actual welding process, and that for analysis of the protective gas (8), protective gas (8) is caused to escape the gas nozzle (27) as is the case in a welding process, and that at least one gas property of the protective gas (8) is measured with at least one sensor (31) and evaluated in an evaluation unit (32) connected to the sensor (31) so that the escape and effect of the protective gas (8) in case of an actual welding process will be simulated, **characterized in that** the oxygen portion of the protective gas (8) is measured, and evaluated, as the gas property of the protective gas (8), and conclusions are automatically drawn as to the impurities of the wearing parts of the torch (10).

7. The measuring method according to claim 6, **characterized in that** the effect of the protective gas (8) is detected in the center of a protective-gas atmosphere (28), wherein the center point of a sensor (31) is positioned to be in an elongated axis (41) of the torch (10) and/or a welding wire (13) extending therein for measuring the gas properties.

8. The measuring method according to claim 6 or 7, **characterized in that** the effect of the protective gas (8) is detected in the rim region of a protective-gas atmosphere (28) by measuring at least one gas property of the protective gas (8) by means of at least one sensor (31).

9. The measuring method according to claim 8, **characterized in that** the torch (10) is moved about a sensor (31) in a substantially circular manner, wherein the center point of the circular movement is formed by the position of the sensor (31).

10. The measuring method according to any one of claims 6 to 9, **characterized in that** a sensor (31) is used for measuring several gas properties.

11. The measuring method according to any one of claims 6 to 9, **characterized in that** a separate sensor (31) is used for each property of the protective gas (8).

12. The measuring method according to any one of claims 6 to 11, **characterized in that** the currently-detected gas properties are compared to those in a memory of the evaluation unit (32) present in the form of deposited and welding-process-adapted reference values.

13. The measuring method according to claim 12, **characterized in that** a starting point of the torch (10), the so-called tool center point (TCP), is controlled on basis of the interrelationship between the position of the torch (10), the protective-gas effect measured, and the corresponding reference values.

14. The measuring method according to any one of claims 6 to 13, **characterized in that** the use of a correct gas nozzle (27) can be concluded automatically after a change of wearing parts by measuring, and evaluating, at least the gas pressure and the rate of the protective gas (8).

15. The measuring method according to any one of claims 6 to 14, **characterized in that** conclusions are drawn automatically as to the pores in the weld to be welded and to the welded weld by measuring the degree of humidity of the protective gas (8).

## Revendications

1. Dispositif de mesure d'un gaz de protection (8) utilisé lors d'un processus de soudure à arc électrique, en analysant un gaz de protection (8) sortant d'une buse de gaz (27) d'un chalumeau (10), moyennant quoi, pour l'analyse du gaz de protection, au moins un capteur (31) est disposé dans un dispositif de mesure externe (30), l'au moins un capteur (31) étant positionné à une distance (38) par rapport au chalumeau (10) correspondant, dans un processus de soudure réel, globalement à la distance entre le chalumeau (10) et une pièce (16), de façon à ce que la sortie et l'action du gaz de protection (8) puissent être simulées comme lors d'un processus de soudure réel et de façon à ce que, pour l'évaluation de l'état des pièces d'usure du chalumeau (10), au moins un capteur (31) soit prévu pour la mesure d'au moins une propriété du gaz de protection (8) et soit relié à une unité d'analyse (32) et, par l'intermédiaire de celle-ci, avec un dispositif de soudure (1), **caractérisé en ce qu'**au moins un capteur (31) est constitué d'un capteur à oxygène (31).

2. Dispositif de mesure selon la revendication 1, **caractérisé en ce que** le dispositif de mesure (30) est combiné avec au moins un capteur (31) pour la mesure du gaz de protection avec un dispositif de nettoyage (33) pour les pièces d'usure du chalumeau (10).

3. Dispositif de mesure selon la revendication 1 ou 2, **caractérisé en ce qu'**au moins un capteur (31) est disposé de manière mobile dans le dispositif de mesure (30).

4. Dispositif de mesure selon l'une des revendications 1 à 3, **caractérisé en ce que**, autour d'un capteur (31) au moins, est disposé un dispositif (42), de préférence circulaire, pour le guidage de l'air comprimé (43).

5. Dispositif de mesure selon l'une des revendications 1 à 4, **caractérisé en ce que**, autour d'au moins un capteur (31), se trouve au moins une ouverture d'aspiration (49), qui est reliée à un dispositif (50) pour l'aspiration du gaz de protection (8).

6. Procédé de mesure d'un gaz de protection (8) utilisé lors d'un processus de soudure à arc électrique, dans lequel le gaz de protection (8) sortant d'une buse de gaz (27) d'un chalumeau (10) est analysé, le chalumeau (10) étant positionné au-dessus d'un dispositif de mesure externe (30) avec au moins un capteur (31) pour l'analyse du gaz de protection, la distance (38) entre le chalumeau (10) et au moins un capteur (31) étant choisie de façon à correspondre globalement à une distance (38) entre le chalumeau (10) et une pièce (16) lors d'un processus de soudure réel et, pour l'analyse, le gaz de protection (8) étant expulsé de la buse de gaz (27), comme lors d'un processus de soudure, et avec au moins un capteur, au moins une propriété du gaz de protection (8) étant mesurée et analysée dans une unité d'analyse (32) reliée au capteur (31), de façon à ce que la sortie et l'action du gaz de protection (8) soient simulées comme lors d'un processus de soudure réel, **caractérisé en ce que** la propriété du gaz de protection (8) mesurée et analysée est la teneur en oxygène du gaz de protection (8) et la présence d'impuretés sur les pièces d'usure du chalumeau (10) en est automatiquement déduite.

7. Procédé de mesure selon la revendication 6, **caractérisé en ce que** l'action du gaz de protection (8) est déterminée au centre d'une enveloppe de gaz de protection (28), moyennant quoi, pour la mesure des propriétés du gaz, le centre d'un capteur (31) est positionné dans un axe prolongé (41) du chalumeau (10) ou d'un fil de soudure (13) s'étendant à l'intérieur de celui-ci.

8. Procédé de mesure selon la revendication 6 ou 7, **caractérisé en ce que** l'action du gaz de protection (8) est déterminée dans la zone de bord d'une enveloppe de gaz de protection (28) en mesurant au moins une propriété du gaz de protection (8) par au moins un capteur (31).

9. Procédé de mesure selon la revendication 8, **caractérisé en ce que** le chalumeau (10) est déplacé de manière globalement circulaire autour d'un capteur (31), le centre du déplacement circulaire étant donné par la position du capteur (31).

10. Procédé de mesure selon l'une des revendications 6 à 9, **caractérisé en ce qu'**un capteur (31) est utilisé pour mesurer plusieurs propriétés du gaz.

11. Procédé de mesure selon l'une des revendications 6 à 9, **caractérisé en ce que**, pour chaque propriété du gaz de protection (8), un capteur (31) différent est utilisé.

12. Procédé de mesure selon l'une des revendications 6 à 11, **caractérisé en ce que** les propriétés du gaz actuellement déterminées sont comparées avec celles qui sont enregistrées dans une mémoire de l'unité d'analyse (32) sous la forme de valeurs de référence enregistrées et adaptées au processus de soudure.

13. Procédé de mesure selon la revendication 12, **caractérisé en ce qu'**un point de départ du chalumeau (10), appellé Tool Center Point (TCP), est contrôlé sur la base de la relation entre la position du chalumeau (10), l'action mesurée du gaz de protection et les valeurs de référence correspondantes.

14. Procédé de mesure selon l'une des revendications 6 à 13, **caractérisé en ce que** l'utilisation d'une buse de gaz (27) correcte après un changement de pièce d'usure peut automatiquement être déduite en mesurant et en analysant au moins la pression et la vitesse du gaz de protection (8).

15. Procédé de mesure selon l'une des revendications 6 à 14, **caractérisé en ce que** la présence de pores dans le cordon de soudure à réaliser et dans le cordon de soudure réalisé peut être déduite en mesurant la teneur en humidité du gaz de protection (8).
